# EUROPEAN PATENT APPLICATION

(11) **EP 4 520 396 A1**
(43) Date of publication of application: **12.03.2025**
(21) Application number: 23306474.0
(22) Date of filing: 05.09.2023
(51) Int. Cl.: A61P 35/00, C07D 401/14, A61K 31/4709

(54) **CRYSTALLINE FREEBASE FORM OF 2-(4-CHLOROBENZYLAMINO)-4-(4-TERT-BUTYLAMINOPIPERIDIN-1-YL)-QUINOLINE AND USES THEREOF**

(71) Applicant: Genoscience Pharma, 13006 Marseille (FR)
(72) Inventor: COURCAMBECK, Jérôme, Marseille (FR); MENUT, Agnès, Marseille (FR); BESTION, Eloïne, Marseille (FR); HALFON, Philippe, Marseille (FR)
(74) Representative: Cabinet Becker et Associés

(57) **Abstract**

The present invention concerns a particular crystalline freebase form of 2-(4-chlorobenzylamino)-4-(4-*tert-*butylaminopiperidin-1-yl)-quinoline. It also relates to a pharmaceutical composition and a kit of parts comprising such a crystalline freebase form and their use in treating and/or preventing cancers, fibrosis, autophagy and cathepsins B (CTSB), L (CTSL) and D (CTSD) related diseases.

## Description

### TECHNICAL FIELD

The present invention belongs to the general medical field and, more particularly, to the field of treatment and/or prevention of various diseases, conditions, disorders and troubles such as, for example, cancers, fibrosis, autophagy and cathepsins B (CTSB), L (CTSL) and D (CTSD) related diseases.

Indeed the present invention concerns a crystal form of a particular 2-primary amino-4-secondary amino-quinoline derivative i.e. a crystal freebase form of 2-(4-chlorobenzylamino)-4-(4-i-eri--butylaminopiperidin-1-yl)-quinoline.

The present invention is also related to a pharmaceutical composition and kit comprising such a compound for use in treating and/or preventing cancers, fibrosis, autophagy and cathepsins B (CTSB), L (CTSL) and D (CTSD) related diseases.

### STATE OF PRIOR ART

The International application WO 2016/067112 A1 discloses novel 2-primary amino-4-secondary amino-quinoline derivatives useful in the treatment and prevention of proliferative neoplastic and non-neoplastic diseases.

These derivatives also present preventive and therapeutic activities in other diseases, conditions, troubles or disorders selected in the group consisting of fibrosis, autophagy and cathepsins B (CTSB), L (CTSL) and D (CTSD) related diseases. The international application WO 2020/048694 A1 relates to such therapeutic applications.

Amongst the 2-primary amino-4-secondary amino-quinoline derivatives presented in the international applications WO 2016/067112 A1 and WO 2020/048694 A1, there is 2-(4-chlorobenzylamino)-4-(4-tert-butylaminopiperidin-1-yl)-quinoline of formula (I-b) as defined in these applications:

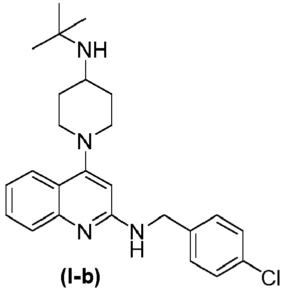

When preparing pharmaceutical compositions and formulations for particular uses, it is highly desirable to have a crystalline form of the therapeutic agent that presents at least one of the following properties: being not hygroscopic, having a relatively high melting point and having a relatively high stability upon storage or ageing.

Indeed moisture absorption may lead to excessive wetting of therapeutic compounds causing their difficult handling during manufacturing. When preparing solid dosage forms containing hygroscopic therapeutic compounds, some formulation strategies have to be developed in order to tackle hygroscopicity issues; these strategies include, for example, film coating, encapsulation and co-processing with excipients. Concerning the melting point, having a high melting point allows the therapeutic compounds to be micronized without significant crystalline alteration.

The inventors have therefore set themselves the goal of proposing a 2-primary amino-4-secondary amino-quinoline derivative having a crystalline form and with at least one of the above properties and advantageously being non-hygroscopic and with high melting point. Having a crystalline form allows advantageously to produce active pharmaceutical ingredient with high purity, ease of handling, drying and storage. The above properties are advantageously required for pharmaceutical industrial manufacturing.

### SUMMARY OF THE INVENTION

The present invention makes it possible to achieve the goal set by the inventors. Indeed the inventors have identified a solid form of 2-(4-chlorobenzylamino)-4-(4-tert-butylaminopiperidin-1-yl)-quinoline which:
- is not hygroscopic at all and does not convert into any hydrated form, even when submitted to high relative humidity (see hereinafter Dynamic water vapor sorption (DVS) analysis),
- has a high melting point (+172°C, see hereinafter Differential Scanning Calorimetry (DSC) analysis) with no degradation and significant mass loss before melting (see hereinafter ThermoGravimetric Analysis (TGA)), and
- is chemically and physically stable as a bulk powder, at least 1-month after storage both at +40°C/75% relative humidity (RH) and at +60°C (see hereinafter).

In addition, this form is crystalline and is obtained by re-crystallization in at least one of the following solvents: ethanol, acetone, methyl ethyl ketone, ethyl acetate, propanol-1, isopropyl acetate and propanol-2. These 7 solvents are solvents of ICH Class 3 (related to ICH guideline Q3C (R8) on impurities: guideline for residual solvents) which means that they are less toxic and of lower risk to human health. This represents an additional advantage of the crystalline form of 2-(4-chlorobenzylamino)-4-(4-tert-butylaminopiperidin-1-yl)-quinoline subject-matter of the present invention which is also designated as "crystalline freebase Form I" or "crystalline Form I" in what follows.

Thus the present invention concerns a particular crystalline freebase form of 2-(4-chlorobenzylamino)-4-(4-i-eri--butylaminopiperidin-1-yl)-quinoline.

As is well known in the field of X-ray powder diffraction (XRPD), relative peak heights of XRPD spectra are dependent on a number of factors relating to sample preparation and instrument geometry, while peak positions are relatively insensitive to experimental details. XRPD patterns for the crystalline freebase Form I were obtained as set forth in hereinafter paragraph II.1. Thus, the crystalline compound of the present invention is characterized by a XRPD pattern having certain peak positions.

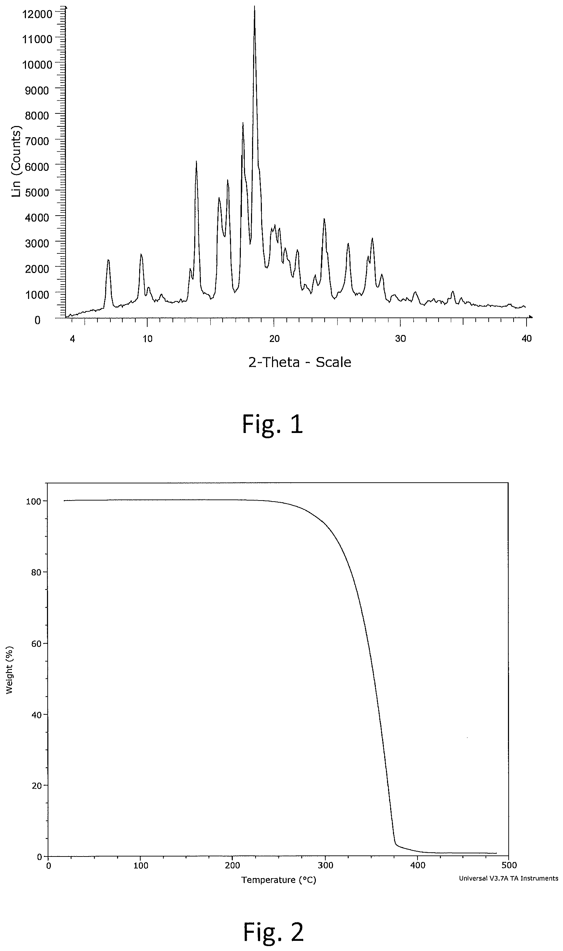

The present invention concerns a particular crystalline freebase form of 2-(4-chlorobenzylamino)-4-(4-tert-butylaminopiperidin-1-yl)-quinoline characterized by a X-ray powder diffraction pattern comprising the diffraction peaks at 2θ values of 13.9±0.1, 16.4±0.1, 17.5±0.1, 17.9±0.1, 18.5±0.1, and 18.8±0.1 (Figure 1).

Advantageously, the X-ray powder diffraction pattern of the crystalline freebase Form I further comprises at least one diffraction peak at 2θ values selected in the group consisting of 9.5±0.1, 15.7±0.1, 19.8±0.1, 20.0±0.1, 20.4±0.1, 20.9±0.1, 21.9±0.1, 24.0±0.1, 24.2±0.1, 25.8±0.1, and 27.8±0.1 (Figure 1). In particular, the X-ray powder diffraction pattern of the crystalline freebase Form I comprises 1, 2, 3, 4, 5, 6, 7, 8, 9, 10 and 11 of these peaks. More particularly, the X-ray powder diffraction pattern of the crystalline freebase Form I comprises the diffraction peaks at 2θ values of 9.5±0.1, 13.9±0.1, 15.7±0.1, 16.4±0.1, 17.5±0.1, 17.9±0.1, 18.5±0.1, and 18.8±0.1, 19.8±0.1, 20.0±0.1, 20.4±0.1, 20.9±0.1, 21.9±0.1, 24.0±0.1, 24.2±0.1, 25.8±0.1, and 27.8±0.1 (Figure 1).

Advantageously, the X-ray powder diffraction pattern of the crystalline freebase Form I further comprises at least one diffraction peak at 2θ values selected in the group consisting of 6.810.1, 13.4±0.1, 21.2±0.1, 23.3±0.1, 27.4±0.1, and 28.6±0.1. In particular, the X-ray powder diffraction pattern of the crystalline freebase Form I comprises 1, 2, 3, 4, 5, and 6 of these peaks. More particularly, the X-ray powder diffraction pattern of the crystalline freebase Form I comprises the diffraction peaks at 2θ values of 6.810.1, 9.5±0.1, 13.4±0.1, 13.9±0.1, 15.7±0.1, 16.4±0.1, 17.5±0.1, 17.9±0.1, 18.5±0.1, and 18.8±0.1, 19.8±0.1, 20.0±0.1, 20.4±0.1, 20.9±0.1, 21.2±0.1, 21.910.1, 23.3±0.1, 24.0±0.1, 24.2±0.1, 25.8±0.1, 27.4±0.1, 27.8±0.1, and 28.6±0.1.

Advantageously, the XRPD pattern of the crystalline freebase Form I is a XRPD pattern in which the peak positions are substantially in accordance with those shown in Figure 1. In particular, the XRPD pattern of the crystalline freebase Form I is as shown in Figure 1.

In yet another embodiment, the crystalline freebase Form I of the present invention is characterized by a thermogravimetric analysis (TGA) performed as described in hereinafter paragraph II.2. Thus, in one embodiment, a crystalline freebase is characterized by its TGA trace. Advantageously, the crystalline freebase Form I of the present invention is characterized by the thermogravimetric analysis (TGA) trace of Figure 2.

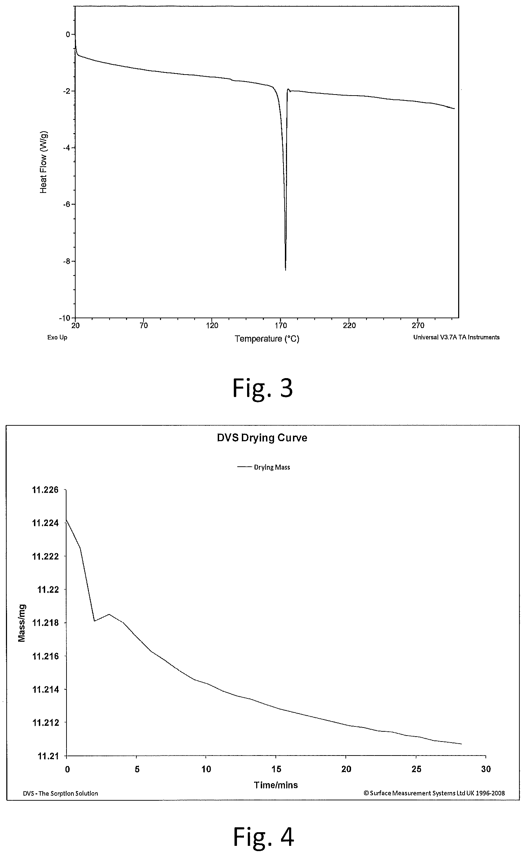

In yet another embodiment, the crystalline freebase Form I of the present invention is characterized by a differential scanning calorimetry (DSC) thermogram obtained as set forth in hereinafter paragraph II.2. The melting point reported herein is estimated on the basis of the melt onset registered during DSC analysis. Advantageously, the crystalline freebase Form I of the present invention is characterized by its DSC thermograph. In particular, the crystalline freebase Form I is characterized by a DSC thermograph which shows a melting point of about +172°C, as described in Figure 3.

In yet another embodiment, the present invention provides a process for preparing the crystalline freebase Form I as previously defined, the process comprising the steps of:
a) mixing 2-(4-chlorobenzylamino)-4-(4-tert-butylaminopiperidin-1-yl)-quinoline (compound I-b) with a solvent selected from the group consisting of ethanol, acetone, methyl ethyl ketone, ethyl acetate, propanol-1, isopropyl acetate and propanol-2;
b) heating the mixture to refluxing conditions or until complete dissolution of 2-(4-chlorobenzylamino)-4-(4-*tert*-butylaminopiperidin-1-yl)-quinoline;
c) cooling the mixture to a temperature below or equal to room temperature (22°C ± 5°C) and
d) isolating the crystalline freebase Form I from the mixture.

In a preferred embodiment, the suitable solvents which can be used in step a) of the preparation method of the crystalline freebase Form I is selected in the group consisting of ethanol, ethyl acetate, and propanol-2.

In yet another embodiment, at step a) of the preparation method of the crystalline freebase Form I, from 2 to 7 volumes of solvent is used by volume of compound I-b.

In yet another embodiment, during step c) of the preparation process of the present invention, crystalline freebase Form I can be added to the mixture. This seeding is preferably performed at the crystallization temperature of crystalline freebase Form I.

The cooling at step c) of the preparation according to the present invention is performed using a controlled temperature ramp and can last from 1 h to overnight (i.e. between 12 h and 15 h).

The isolation at step d) of the preparation according to the present invention can consists in filtering the mixture whereby the crystalline freebase Form I is obtained, in washing the crystalline freebase Form I with the same solvent as the one implemented in step a) and in drying the crystalline freebase Form I.

The 2-primary amino-4-secondary amino-quinoline derivatives and in particular the 2-(4-chlorobenzylamino)-4-(4-tert-butylaminopiperidin-1-yl)-quinoline of formula (I-b) can be used for treating and/or preventing cancers, fibrosis, autophagy and cathepsins B (CTSB), L (CTSL) and D (CTSD) related diseases. Accordingly, a crystalline freebase of the compound of formula (I-b), i.e. the crystalline freebase Form I, is expected to have the same activity independently of its crystalline or amorphous form and independently of its pharmaceutical formulation in solid form and even more in liquid form. A crystalline freebase of the compound of formula (I-b) is intended to be used in medicine and in particular for use in preventing and/or treating at least one disease, condition, trouble or disorder selected from cancers, fibrosis, autophagy and cathepsins B (CTSB), L (CTSL) and D (CTSD) related diseases.

Thus, the crystalline freebase Form I of the present invention may be formulated as a pharmaceutical composition (or formulation). Consequently, the present invention also relates to a pharmaceutical composition or formulation comprising, as active ingredient, the crystalline freebase Form I as previously defined. However, it will be understood by those skilled in the art that, once the crystalline freebase form I of the present invention has been formulated, it may no longer be in crystalline form, i.e., the crystalline freebase may be dissolved in a suitable carrier such as a pharmaceutically acceptable vehicle.

Typically, the pharmaceutical composition implemented in the present invention comprises, as active ingredient, the crystalline freebase Form I as previously defined and at least one pharmaceutically acceptable vehicle.

The expression "pharmaceutically acceptable vehicle" as used herein is meant any substance which is added to the active ingredient implemented in the present invention to promote its transport, avoid its substantial degradation in said composition and/or increase its half-life. Advantageously, such a pharmaceutically acceptable vehicle is sterile and non-pyrogenic and refers to molecular entities and compositions that do not produce an adverse, allergic or other untoward reaction when administered to an animal, in particular, a mammal, especially a human, as appropriate. It is chosen depending on the type of application of the pharmaceutical composition of the present invention and in particular as a function of its administration mode. Advantageously, a pharmaceutically acceptable vehicle refers to a non-toxic, solid, semi-solid or liquid carrier, filler, diluent, additive, excipient, buffer, encapsulating material or formulation auxiliary of any type.

The pharmaceutical composition or formulation of the present invention can be administered by the systemic route; by the parenteral route, for example by intravenous, intra-arterial, intraperitoneal, intrathecal, intraventricular, intrasternal, intracranial, intramuscular or sub-cutaneous routes; by the topical route; by the ocular route; by the oral route; and by the mucosal route such as the buccal, nasal, intranasal, rectal and vaginal routes. The pharmaceutical composition or formulation of the present invention can also be administered by inhalation aerosol technology.

As a solid pharmaceutical composition for oral administration implemented in the present invention, tablets, pills, powders, granules or capsules can be used where the active ingredient is mixed with one or more conventionally used inert diluents such as, for example, starch, calcium carbonate, sucrose, lactose, mannitol, cellulose esters, microcrystalline cellulose, polyethylene glycol, polypropylene glycol or gelatin, and possibly other substances such as, for example, a lubricant which may be magnesium stearate or talc, a colorant, or a coating.

As a liquid pharmaceutical composition for oral or ocular administration implemented in the present invention, pharmaceutically acceptable, suspensions, solutions, emulsions, syrups containing conventionally used inert diluents, and possibly other substances such as, for example, wetting products, humectants, sweetening agents, flavoring agents, preservatives, or thickeners can be used.

The sterile pharmaceutical composition for parenteral administration implemented in the present invention can be sterilized, aqueous or non-aqueous solution, suspension or emulsion. As a solvent or vehicle, water, propylene-glycol, polyethylene glycol, plant oils, injectable ester like ethyl oleate or other suitable organic solvents can be used. This composition can also contain adjuvants, such as wetting agents, isotonising agents or emulsifiers.

The pharmaceutical composition for topic administration implemented in the present invention can be, for example, creams, lotions, oral sprays, nose or eye drops or aerosol.

In addition, in the pharmaceutical composition according to the present invention, the crystalline freebase Form I may be formulated or co- formulated in nanoparticles.

In a particular embodiment, these nanoparticles may comprise a polymeric biodegradable composition. The polymer implemented in this composition may be based on poly(DL-Lactic-co-glycolic acid) having molecular weight from 7 to 240 kDa; or a copolymer of polylactic acid (PLA) and polyglycolic acid (PGA) where the molecular ratio is between 95:5 and 50:50.

In a particular embodiment, these nanoparticles may comprise lysosomal biodegradable composition, a biocompatible polymer or copolymer or a liposomal formulation.

Whatever embodiment considered, the nanoparticles may be associated covalently or non-covalently with a polyethylene glycol (PEG) and/or may have an average size of from about 80 to about 600 nm.

Additional information on these nanoparticles, methods and materials to prepare them may be found in the International application WO 2016/067112 A1.

In a particular embodiment, the pharmaceutical composition implemented in the present invention further comprises at least one additional therapeutic agent.

Thus, the pharmaceutical composition implemented in the present invention comprises or consists of (i) the crystalline freebase Form I, (ii) at least one pharmaceutically acceptable vehicle and (iii) at least one additional therapeutic agent.

Typically, the additional therapeutic agent(s) present in the pharmaceutical composition implemented in the present invention may be therapeutic agent(s) already used in the prevention and/or the treatment of at least one disease, condition, trouble or disorder selected from the group consisting of cancers, fibrosis, autophagy and cathepsins B (CTSB), L (CTSL) and D (CTSD) related diseases.

The additional therapeutic agent(s) may be selected in the group consisting of:
- anti-neoplastic agents and, in particular, the ones cited from page 8, line 9 to page 10, line 2 of the International application WO 2016/067112 A1,
- antifibrotic agents and agents used to palliate, ameliorate, stabilize, reverse, slow or delay the progression of the fibrotic state,
- anti-autophagic agents and agents used to palliate, ameliorate, stabilize, reverse, slow or delay the progression of the autophagic disease state and/or related diseases and agents used to inhibit the autophagy flux,
- inhibitors of cathepsins B (CTSB), L (CTSL) and/or D (CTSD) and agents used to palliate, ameliorate, stabilize, reverse, slow or delay the progression of cathepsins B (CTSB), L (CTSL) and/or D (CTSD) related diseases.

Moreover, the pharmaceutical composition implemented in the present invention may further comprise at least one additional active agent selected from the agents listed from page 274, line 25 to page 280, line 2 of the International application WO 2020/048694 A1.

In a particular embodiment, the pharmaceutical composition implemented in the present invention comprises at least two or at least three additional therapeutic agents selected in a same list or in different lists amongst the previously defined lists and/or at least two or at least three additional active agents selected in a same list or in different lists amongst the lists disclosed from page 274, line 25 to page 280, line 2 of the International application WO 2020/048694 A1.

A dosage of the pharmaceutical composition implemented in the present invention needs to be a pharmaceutically effective amount. The "pharmaceutically effective amount" means an amount enough to prevent or treat diseases at a reasonable benefit/risk ratio applicable to medical treatment, and a level of effective dose may be variously selected by those skilled in the art according to factors such as, for example, a formulation method, a patient's condition including weight, gender and age, a degree of disease, a drug form, an administration route, an administration period if this administration implements single or fractionated doses, an excretion rate and reaction sensitivity. The effective amount may vary depending on a route of disposal, a use of excipients and possibility of being used with other therapeutic agent(s) and/or other active agent(s), as recognized by those skilled in the art.

The present invention also concerns a kit of parts comprising or consisting of:
a) the crystalline freebase Form I or a pharmaceutical composition comprising it as previously defined, and
b) at least one additional therapeutic agent or additional active agent, in particular such as previously defined or a pharmaceutical composition comprising at least one additional therapeutic agent or additional active agent.

A kit of parts can also be defined as a combination of the elements a) and b) as above defined.

Such a kit of parts is of particular interest in the present invention when the crystalline freebase Form I and the at least one additional therapeutic agent or additional active agent cannot be formulated in the same pharmaceutical composition and/or when the crystalline freebase Form I or the pharmaceutical composition comprising it and the at least one additional therapeutic agent or the at least one additional active agent or the pharmaceutical composition comprising it are to be administrated separately or sequentially.

Thus, the kit of parts as implemented in the present invention can be used simultaneously, separately or sequentially and in particular for preventing and/or treating at least one disease, condition, trouble or disorder selected from cancers, fibrosis, autophagy and cathepsins B (CTSB), L (CTSL) and D (CTSD) related diseases.

As used herein, a simultaneous use means that the element a) as above defined is administrated as the same time as the element b) as above defined to a patient. The zone of administration at the level of the patient and thus the administration route can be identical or different.

As used herein, a separate or sequential use means that the elements a) and b) as above defined are administrated separately or sequentially provided that the time period during which the element a) exerts its pharmacological effects on the patient and the time period during which the element b) exerts its pharmacological effects on the patient at least partially intersect.

As used herein, a "patient", a "patient in need", a "subject" and a "subject in need" mean either a subject at risk of developing at least one disease, condition, trouble or disorder selected from cancers, fibrosis, autophagy and cathepsins B (CTSB), L (CTSL) and D (CTSD) related diseases or a subject already suffering from at least one disease, condition, trouble or disorder selected from cancers, fibrosis, autophagy and cathepsins B (CTSB), L (CTSL) and D (CTSD) related diseases. As used herein, the terms "subject" and "subjects" refer to an animal (e.g. birds, reptiles, and mammals), Mammals are any member of the Mammalian class, including but not limited to humans, non-human primates such as chimpanzees, orangutan, gorilla, and other apes and monkey species; farm animals such as cattle, horses, camels, sheep, goats, swine; domestic animals such as rabbits, dogs, and cats; laboratory animals including rodents, such as rats, mice and guinea pigs, and the like. Preferably a mammal include a non-primate mammal (e.g. a camel, donkey, zebra, cow, pig, horse, goat, sheep, cat, dog, rat, and mouse) and a primate (e.g. a monkey, chimpanzee, and a human). Examples of non-mammals include, but are not limited to, birds, fish and the like. In some embodiments of the methods and compositions provided herein, the subject is a mammal. In some preferred embodiments, the subject is a human.

In addition, the crystalline freebase Form I as previously defined, the pharmaceutical composition as previously defined and/or the kit of parts as previously defined is implemented for use in preventing and/or treating a proliferative and/or neoplastic disease. In particular, this proliferative and/or neoplastic disease may be selected in the group consisting of: carcinoma; head, eye, kidney, bladder, liver, lung, nasopharyngeal, neck, ovary, breast, cervix, pancreas, prostate, or stomach cancers; a leukemia (e.g. acute myelogenous leukemia, acute lymphocytic leukemia, acute promyelocytic leukemia (APL), acute T-cell lymphoblastic leukemia, adult T-cell leukemia, basophilic leukemia, eosinophilic leukemia, granulocytic leukemia, hairy cell leukemia, leukopenic leukemia, lymphatic leukemia, lymphoblastic leukemia, lymphocytic leukemia, megakaryocytic leukemia, micromyeloblastic leukemia, monocytic leukemia, neutrophilic leukemia and stem cell leukemia); a malignant lymphoma; a malignant melanoma; myeloproliferative diseases; a sarcoma; a tumor of the central nervous system; a germ-line tumor; testicular cancer; thyroid cancer; astrocytoma; esophageal cancer; colon cancer, and a mixed type of neoplasia.

In some embodiments, the crystalline freebase Form I as previously defined, the pharmaceutical composition as previously defined and/or the kit of parts as previously defined is implemented for use in preventing and/or treating liver cancer related diseases. In other words, the proliferative and/or the neoplastic disease is a liver cancer related disease.

In a particular embodiment, such a cancer related disease is a lung cancer.

In another particular embodiment, such a cancer related disease is an ovary cancer.

In another particular embodiment, such a cancer related disease is a colon cancer.

In another particular embodiment, such a cancer related disease is a pancreas cancer.

In another particular embodiment, such a liver cancer related disease is a hepatocarcinoma, a hepatoblastoma or a cholangiocarcinoma.

In another particular embodiment, such a liver cancer related disease is a hepatocarcinoma.

In another particular embodiment, such a liver cancer related disease is a cholangiocarcinoma and, more particularly, is an intrahepatic cholangiocarcinoma or an extrahepatic cholangiocarcinoma.

The crystalline freebase Form I as previously defined, the pharmaceutical composition as previously defined and/or the kit of parts as previously defined may also be implemented for use in inhibiting the growth or differentiation of a Cancer Stem Cell (CSC), a tumor initiating cell, a mesenchymal-like cell associated with cancer, a mesenchymal cancerous cell, or a mesenchymal cell.

The crystalline freebase Form I as previously defined, the pharmaceutical composition as previously defined and/or the kit of parts as previously defined may also be implemented for use in treating and/or decreasing and/or preventing fibrosis and/or fibrosis related diseases.

This embodiment may consist in treating a fibrotic diseases and conditions selected in the group consisting of fibrotic skin diseases and conditions, such as scleroderma, subcutaneous scarring such as keloids, adhesions, hypertrophic scarring or cosmetic scarring; lung fibrotic diseases and conditions, such as pulmonary fibrosis including idiopathic pulmonary fibrosis (IPF), non-idiopathic pulmonary fibrosis interstitial pneumonias, interstitial lung disease (ILD), idiopathic interstitial lung disease, pulmonary hypertension, chronic obstructive pulmonary disease (COPD) or sarcoidosis; liver fibrotic diseases and conditions, such as cirrhosis of the liver, hemochromatosis, hepatitis B virus infection, hepatitis C virus infection, hepatitis delta virus infection, autoimmune hepatitis, Wilson's disease, Budd-Chiari syndrome alpha-1-antitrypsin deficiency, primary biliary cirrhosis, primary sclerosing chlolangitis, biliary atresia, biliary obstruction, fibrosis of the liver, Non-alcoholic fatty liver disease (NAFLD) and Non-alcoholic steatohepatitis (NASH), alcoholic steatohepatitis, alcohol abuse, focal fatty liver, cholestatic liver disease, cirrhosis and non-cirrhosis portal hypertension, portal vein thrombosis, liver veno-occlusive disease, congenital hepatitis fibrosis, heart failure, and diabetes; kidney fibrotic diseases and conditions, such as progressive kidney disease glomerulonephritis, glomerular disease, kidney fibrosis and diabetic nephropathy; heart fibrotic diseases and conditions such as heart failure due to ischaemic heart disease, valvular heart disease and hypertensive heart disease, diabetic cardiomyopathy and hypertension; intestinal fibrotic disease and conditions including bowel fibrosis, colon fibrosis, small intestine fibrosis, inflammatory bowel disease, ulcerative colitis, Crohn's disease, infection with Schistosoma mansoni; eye fibrotic disease and conditions such as diabetic retinopathy and other fibrotic diseases and conditions such as nerve system related fibrosis; mediastinum related fibrosis; retroperitoneum related fibrosis; joint and tendon (arthrofibrosis); or as an adjuvant or anti-fibrotic in pancreatic diseases such to increase chemotherapeutic drug penetration by reducing the density of the connective tissue stroma;

This embodiment may also consist in treating a liver fibrotic diseases and conditions such as cirrhosis of the liver, hemochromatosis, hepatitis B virus infection, hepatitis C virus infection, hepatitis delta virus infection, autoimmune hepatitis, Wilson's disease, Budd-Chiari syndrome alpha-1-antitrypsin deficiency, primary biliary cirrhosis, primary sclerosing chlolangitis, biliary atresia, biliary obstruction, fibrosis of the liver, Non-alcoholic fatty liver disease (NAFLD) and Non-alcoholic steatohepatitis (NASH), alcoholic steatohepatitis, alcohol abuse, focal fatty liver, cholestatic liver disease, cirrhosis and non-cirrhosis portal hypertension, portal vein thrombosis, liver veno-occlusive disease, congenital hepatitis fibrosis, heart failure, and diabetes.

This embodiment may also consist in treating lung fibrotic diseases and conditions, such as pulmonary fibrosis including idiopathic pulmonary fibrosis (IPF), non-idiopathic pulmonary fibrosis interstitial pneumonias, interstitial lung disease (ILD), idiopathic interstitial lung disease, pulmonary hypertension, chronic obstructive pulmonary disease (COPD) or sarcoidosis.

This embodiment may also consist in treating kidney fibrotic diseases and conditions, such as progressive kidney disease glomerulonephritis, glomerular disease, kidney fibrosis and diabetic nephropathy.

The crystalline freebase Form I as previously defined, the pharmaceutical composition as previously defined and/or the kit of parts as previously defined may be implemented for use in decreasing and/or preventing the autophagy and/or autophagy related diseases and inhibiting autophagy flux related diseases. This embodiment may consist in treating or preventing and/or ameliorating autophagy or hyperautophagy and/or autophagy or hyperautophagy related diseases or diseases characterized by an increased autophagy flux or condition responding favorably to inhibition of autophagy. In particular, the autophagy and/or autophagy related diseases or disorders may be selected from rheumatoid arthritis (RA), malaria, antiphospholipid antibody syndrome, lupus, chronic urticarial, Gougerot-Sjogren syndrome, Guillain-Barre disease, Alzheimer's or Parkinson's diseases, eosinophilic airway inflammation, regeneration of fat tissue, psoriasis, multiple sclerosis (MS), muscular dystrophy (MD), myopathies, asthma, chronic pulmonary obstructive disorder (COPD), Crohn's disease (CD), chronic inflammatory demyelinating polyradiculoneuropathy (CIDP), fibromyalgia, diabetes, polymyositis, pulmonary diseases, chronic immune thrombocytopenia (ITP), ulcerative colitis, irritable bowel syndrome, scleroderma, neurodegeneration, heart failure, obesity, sarcopenia, aging, inflammatory disorders, ischemia/reperfusion, lysosomal storage diseases, infectious diseases associated with intracellular pathogens including viruses, bacteria, and parasites such as Trypanosomes.

Then, the crystalline freebase Form I as previously defined, the pharmaceutical composition as previously defined and/or the kit of parts as previously defined may be implemented for use in inhibiting cathepsins B (CTSB), L (CTSL) and/or D (CTSD) and/or treating and/or preventing cathepsins B (CTSB), L (CTSL) and/or D (CTSD) related diseases. This embodiment may consist in treating or preventing and/or ameliorating symptoms of Alzheimer's disease, rheumatism arthritis, inflammatory airway disease, chronic obstructive pulmonary disease, osteoarthritis, pneumocystis carinii, acute pancreatitis, multiple sclerosis, bone and joint disease or disorder in a subject in need thereof, arthritis, inflammatory diseases such as chronic and acute pancreatitis, inflammatory airway disease, and bone and joint disorders, including osteoporosis, osteoarthritis, rheumatoid arthritis, psoriasis, and other autoimmune disorders, liver fibrosis, including liver fibrosis associated with hepatitis B and/or C, all types of steatosis including non-alcoholic steatohepatitis and alcohol-associated steatohepatitis, non-alcoholic fatty liver disease, cirrhosis, autoimmune hepatitis, biliary obstruction, hemochromatosis, alpha-1 antitrypsin deficiency, Wilson's disease, primary biliary cirrhosis, primary sclerosing cholangitis, Budd-Chiari syndrome, portal vein thrombosis, liver veno-occlusive disease, congenital hepatitis fibrosis, heart failure, forms of pulmonary fibrosis including idiopathic pulmonary fibrosis, pathological diagnosis of interstitial pneumonia following lung biopsy, renal fibrosis, cardiac fibrosis, retinal angiogenesis and fibrosis/gliosis in the eye, scleroderma, and systemic sclerosis.

The present invention also concerns a method for preventing and/or treating a proliferative and/or neoplastic disease in a patient in need thereof, wherein said method comprises a step of administrating, to the patient in need thereof, a crystalline freebase Form I such as previously defined, a pharmaceutical composition such as previously defined or a kit of parts such as previously defined.

The present invention also concerns a method for inhibiting the growth or differentiation of a Cancer Stem Cell (CSC), a tumor initiating cell, a mesenchymal-like cell associated with cancer, a mesenchymal cancerous cell, or a mesenchymal cell in a patient in need thereof, wherein said method comprises a step of administrating, to the patient in need thereof, a crystalline freebase Form I such as previously defined, a pharmaceutical composition such as previously defined or a kit of parts such as previously defined.

The present invention also concerns a method for treating and/or decreasing and/or preventing fibrosis and/or a fibrosis related disease in a patient in need thereof, wherein said method comprises a step of administrating, to the patient in need thereof, a crystalline freebase Form I such as previously defined, a pharmaceutical composition such as previously defined or a kit of parts such as previously defined.

The present invention also concerns a method for decreasing and/or preventing the autophagy and/or an autophagy related disease and for inhibiting an autophagy flux related disease in a patient in need thereof, wherein said method comprises a step of administrating, to the patient in need thereof, a crystalline freebase Form I such as previously defined, a pharmaceutical composition such as previously defined or a kit of parts such as previously defined.

The present invention also concerns a method for inhibiting cathepsins B (CTSB), L (CTSL) and/or D (CTSD) and/or for treating and/or preventing a cathepsins B (CTSB), L (CTSL) and/or D (CTSD) related disease in a patient in need thereof, wherein said method comprises a step of administrating, to the patient in need thereof, a crystalline freebase Form I such as previously defined, a pharmaceutical composition such as previously defined or a kit of parts such as previously defined.

As already disclosed for the pharmaceutical composition, the crystalline freebase Form I and the elements a) and b) of the kit of parts as previously defined can be administered by the systemic route; by the parenteral route, for example by intravenous, intra-arterial, intraperitoneal, intrathecal, intraventricular, intrasternal, intracranial, intramuscular or sub-cutaneous route; by the topical route; by the ocular route; by the oral route and by the mucosal route such as the buccal, nasal, intranasal, rectal and vaginal routes. They may also be administered by inhalation aerosol technology i.e. thanks to an aerosol application by buccal way or upper airways for pulmonary use.

In addition, as already disclosed for the pharmaceutical composition, the crystalline freebase Form I and the elements a) and b) of the kit of parts as previously defined are to be administrated in pharmaceutically effective amounts. The definition previously given for pharmaceutical composition applies *mutatis mutandis* to the crystalline freebase Form I thereof and the elements a) and b) of the kit of parts.

Other characteristics and advantages of the present invention will additionally be apparent to the one skilled in the art on reading the examples below, which are given as an illustration and not a limitation, with reference to the attached figures.

### BRIEF DESCRIPTION OF THE FIGURES

Figure 1 presents the XRPD profile for crystalline Form I (example II.1).
Figure 2 presents the TGA profile for crystalline Form I (example II.2).
Figure 3 presents the DSC profile for crystalline Form I (example II.2).
Figure 4 presents the DVS isotherm drying curve at +25°C for crystalline Form I (mass *versus* time) (example II.3).
Figure 5 presents the DVS isotherm sorption plot at +25°C for crystalline Form I: cycle 1 (example II.3).
Figure 6 presents the DVS isotherm sorption plot at +25°C for crystalline Form I: cycle 1 and cycle 2 (example II.3).
Figure 7 presents the overlaid crystalline Form I TGA profiles of a non-stressed sample and of samples stored for 1 month at +60°C and at +40°C/75% RH (example IV.2).
Figure 8 presents the overlaid crystalline Form I DSC profiles of a non-stressed sample (top trace) and of samples stored for 1 month at +60°C (middle trace) and at +40°C/75% RH (bottom trace) (example IV.2).
Figure 9 presents the overlaid XRPD patterns of crystalline Form I for a non-stressed sample (top trace) and samples stored for 1 month at +60°C (middle trace) and at +40°C/75% RH (bottom trace) (example IV.2).

### EXAMPLES

### I. Preparation of the crystalline form of 2-(4-chlorobenzylamino)-4-(4-tert-butylaminopiperidin-1-yl)-quinoline.

### 1.1. Synthesis of 2-(4-chlorobenzylamino)-4-(4-tert-butylaminopiperidin-1-yl)-quinoline.

2-(4-chlorobenzylamino)-4-chloroquinoline is prepared as disclosed in the International applications WO 2016/067112 A1 and WO 2020/048694 A1. To a solution of this compound (1.05 g, 3.46 mmol) and 4-(tert-butylamino)-piperidine 1-2 (0.684 g, 4.38 mmol) in 5 ml of N-Methylpyrrolidone was added N,N-Diisopropylethylamine (0.947 ml, 5.47 mmol) and the mixture was heated for 22h at 140°C. Then, the reaction mixture was cooled to room temperature (22°C ± 5°C), diluted with a 1N NaOH aqueous solution and the resulting mixture was extracted with EtOAc. The combined organic layers were dried over Na₂SO₄, filtered and concentrated under reduced pressure to give a yellow oil. The crude product was purified by flash chromatography (gradient cyclohexane/ethyl acetate from 8/2 to 0/10) to give a yellow solid corresponding to 2-(4-chlorobenzylamino)-4-(4-tert-butylaminopiperidin-1-yl)-quinoline.

### I.2. Re-crystallization of 2-(4-chlorobenzylamino)-4-(4-tert-butylaminopiperidin-1-yl)-quinoline.

The solvents that are used as re-crystallization media are listed in Table 1.

**Table 1 : Solvents used for re-crystallization and their references**

| | **Solvent** | **Abbreviation** | **ICH class** | **Provider** | **Reference** |
|---|---|---|---|---|---|
| **1** | Ethanol | EtOH | 3 | Sigma-Aldrich | 34870 |
| **2** | Acetone | - | 3 | Carlo Erba | 412501 |
| **3** | Methyl Ethyl Ketone | MEK | 3 | Carlo Erba | 462701 |
| **4** | Ethyl Acetate | EtAc | 3 | Carlo Erba | 448251 |
| **5** | Propanol-1 | - | 3 | Sigma-Aldrich | 34871 |
| **6** | isopropyl Acetate | IPAc | 3 | Sigma-Aldrich | 45960 |
| **7** | Propanol-2 | IPA | 3 | Sigma-Aldrich | 34863 |

Prior to launching the slurry experiments, an "apparent solubility" is determined in all ageing media. To do so, to accurately weighed small amounts of 2-(4-chlorobenzylamino)-4-(4-*tert*-butylaminopiperidin-1-yl)-quinoline, aliquots of each solvent are successively added until complete dissolution is observed. The corresponding solubility S is then estimated.

The following Table 2 displays the results of the compound "visual" solubility assessment at 20-25°C that allowed defining the amount of compound to be used in each medium of the micro-crystallization screening.

**Table 2 : Visual solubility results at 20-25°C for 2-(4-chlorobenzylamino)-4-(4-tert-butylaminopiperidin-1-yl)-quinoline**

| **Media** | **Estimated solubility (mg/mL)** | **Solubility** |
|---|---|---|
| **EtOH** | > 9.8 | Medium |
| **Acetone** | > 16.0 | Medium |
| **MEK** | > 2.4 | Low |
| **EtAc** | > 17.1 | Medium |
| **Propanol-1** | > 15.1 | Medium |
| **IPAc** | > 15.2 | Medium |
| **IPA** | > 2.6 | Low |

These 7 media are kept for the polymorphism screen, based on micro-crystallizations experiments by ageing (slurries).

A certain amount of 2-(4-chlorobenzylamino)-4-(4-tert-butylaminopiperidin-1-yl)-quinoline is introduced at room temperature into each solvent. The resulting slurries are allowed to ageing for 6 weeks at sub-ambient temperature, for example, at +4°C, away from light. After this time period, the solid residues are characterized by optical microscopy, XRPD and DSC analysis. Finally, TGA and/or TGA-FTIR and HPLC analyses could be performed on certain samples, chosen from the XRPD and DSC results.

Higher quantities of crystalline freebase Form I of compound I-b can be prepared by charging into a chemical reactor compound I-b and a solvent, preferably ethanol, or acetone, or methyl ethyl ketone, or ethyl acetate, propanol-1, or isopropyl acetate or propanol-2 (from 2 to 10 volumes, e.g. EtOH 7 volumes). The resulting mixture was heated from room temperature to refluxing conditions or until complete dissolution of compound I-b. The temperature of the reaction mixture was then to decrease to room temperature or below the room temperature (e.g. 0°C) using a controlled temperature ramp (e.g. from overnight to 1 hour). At the temperature of the crystallization a typical seeding with form I of compound I-b can be advantageously done. The final product is obtained after filtration, washing with the solvent used for the recrystallization (e.g. 1 to 2 volumes at room temperature or less typically 0°C). The resulting wet solid was then dried to afford the form I of compound I-b.

### II. Characterization of the crystalline form of 2-(4-chlorobenzylamino)-4-(4-tert-butylaminopiperidin-1-yl)-quinoline thus obtained in ethanol (hereinafter crystalline Form 1).

For this characterization, ethanol has been selected as the solvent for preparing the crystalline Form I since it presents an intermediate solubility profile.

### II.1. Freebase crystallinity by XRPD analysis.

### Method: X-ray powder diffraction (XRPD)

X-ray powder diffraction (XRPD) analysis is performed on a Brüker D2 Phaser diffractometer, using a copper anti-cathode, a mono-crystalline silicon sample holder and a position sensitive detector (LynxEye). Instrument operating conditions for X-rays pattern acquisition are described in Table 3.

Powder sample is loaded on a flat mono-crystalline silicon sample holder in a way to avoid preferred orientation (not randomly oriented particles) and to ensure planarity of the specimen surface.

**Table 3 : Instrument operating conditions for X-rays profile acquisition**

| | | | |
|---|---|---|---|
| Temperature | | | Ambient |
| Atmosphere | | | Ambient |
| X-rays generator | - voltage (kV) | | 30 |
| | - intensity (mA) | | 10 |
| X-rays source | - ta rget | | Cu |
| | - emission radiation | Kλ₁ (nm) | 0.15406 |
| | | Kλ₂ (nm) | 0.15444 |
| | | ratio Kλ₂/Kλ₁ | 0.5 |
| | - Kβ filter radiation | | Nickel |
| Slit | - anti-divergence (mm) | | 1 |
| | - Sollers slit (°) | | 2.5 |
| Goniometer | - angular sector analyzed (° for 2θ) | | 4-40 |
| | - step size (° for 2 θ) | | 0.07 |
| Sample holder rotation speed (rpm) | | | 30 |
| Detection | - exposition time per step size of goniometer (s) | | 1 |

### Result: Freebase crystallinity by XRPD analysis

The XRPD profile presented in Figure 1 for crystalline form I evidences an organic powder of good crystallinity with numerous, relatively intense and well-defined diffraction peaks between 6 and 35 in 2-theta. In the experimental conditions used, no significant amorphous fraction is evidenced.

The Table 4 hereinafter presents the list of the different diffraction peaks with their intensity (count per second c.p.s.).

**Table 4: Diffraction peaks (2θ) with their intensity (count per second c.p.s.) for crystalline Form I**

| Angle 2-Thêta (°) | Inter-reticular distance (Å) | Intensity | | Angle 2-Thêta (°) | Inter-reticular distance (Å) | Intensity | |
|---|---|---|---|---|---|---|---|
| | | c.p.s. | % | | | c.p.s. | % |
| 3.8 | 23.0 | 99 | 0.8 | 22.5 | 4.0 | 1271 | 10.4 |
| 4.3 | 20.4 | 112 | 0.9 | 22.7 | 3.9 | 1172 | 9.6 |
| 4.8 | 18.3 | 201 | 1.6 | 23.3 | 3.8 | 1629 | 13.3 |
| 5.1 | 17.3 | 222 | 1.8 | 24.0 | 3.7 | 3837 | 31.4 |
| 5.6 | 15.8 | 278 | 2.3 | 24.2 | 3.7 | 2675 | 21.9 |
| 6.3 | 14.0 | 337 | 2.8 | 25.1 | 3.5 | 979 | 8.0 |
| 6.8 | 12.9 | 2238 | 18.3 | 25.8 | 3.4 | 2884 | 23.6 |
| 7.7 | 11.5 | 515 | 4.2 | 27.4 | 3.2 | 2376 | 19.4 |
| 7.9 | 11.2 | 480 | 3.9 | 27.8 | 3.2 | 3075 | 25.2 |
| 8.3 | 10.6 | 508 | 4.2 | 28.6 | 3.1 | 1674 | 13.7 |
| 8.6 | 10.3 | 626 | 5.1 | 29.5 | 3.0 | 856 | 7.0 |
| 9.5 | 9.3 | 2470 | 20.2 | 30.3 | 3.0 | 707 | 5.8 |
| 10.0 | 8.8 | 1166 | 9.5 | 30.5 | 2.9 | 751 | 6.1 |
| 10.6 | 8.3 | 732 | 6.0 | 30.8 | 2.9 | 669 | 5.5 |
| 11.1 | 8.0 | 883 | 7.2 | 31.2 | 2.9 | 983 | 8.0 |
| 12.2 | 7.2 | 609 | 5.0 | 32.3 | 2.8 | 632 | 5.2 |
| 12.7 | 7.0 | 692 | 5.7 | 32.7 | 2.7 | 708 | 5.8 |
| 13.4 | 6.6 | 1900 | 15.5 | 33.1 | 2.7 | 638 | 5.2 |
| 13.9 | 6.4 | 6126 | 50.1 | 33.3 | 2.7 | 632 | 5.2 |
| 14.5 | 6.1 | 1013 | 8.3 | 33.6 | 2.7 | 580 | 4.7 |
| 15.7 | 5.6 | 4692 | 38.4 | 33.9 | 2.6 | 727 | 5.9 |
| 16.4 | 5.4 | 5390 | 44.1 | 34.2 | 2.6 | 996 | 8.1 |
| 17.5 | 5.1 | 7627 | 62.4 | 34.9 | 2.6 | 738 | 6.0 |
| 17.9 | 5.0 | 5292 | 43.3 | 35.1 | 2.6 | 625 | 5.1 |
| 18.5 | 4.8 | 12225 | 100.0 | 35.4 | 2.5 | 592 | 4.8 |
| 18.8 | 4.7 | 5979 | 48.9 | 35.5 | 2.5 | 633 | 5.2 |
| 19.8 | 4.5 | 3473 | 28.4 | 35.6 | 2.5 | 566 | 4.6 |
| 20.0 | 4.4 | 3604 | 29.5 | 35.8 | 2.5 | 498 | 4.1 |
| 20.4 | 4.3 | 3484 | 28.5 | 37.0 | 2.4 | 468 | 3.8 |
| 20.9 | 4.2 | 2704 | 22.1 | 38.8 | 2.3 | 493 | 4.0 |
| 21.2 | 4.2 | 2383 | 19.5 | 39.8 | 2.3 | 420 | 3.4 |
| 21.9 | 4.1 | 2632 | 21.5 | | | | |

### II.2. Thermal profile TGA and DSC analyses.

### Method: Differential scanning calorimeter (DSC)

Differential scanning calorimeter (DSC) analysis is performed on a Q1000 TA Instruments analyzer. Sample is weighed in an aluminum capsule, which is then crimped and put into the calorimeter oven.

The instrumental operating conditions for DSC profile acquisition are described in Table 5.

**Table 5: Instrument operating conditions for DSC profile acquisition**

| | | |
|---|---|---|
| Heater ramp (°C/min) | | 10 |
| Final temperature (°C) | | +250 or +300°C |
| Carrier gas | - nitrogen | Messer, "Nitrogen quality 5.0" |
| | - flow rate (mL/min) | 50 |

### Method: Thermogravimetric analysis coupled to infrared (TGA-FTIR) analysis

Thermogravimetric analysis is performed on a TA Instruments TGA Hi-Res 2950 apparatus equipped with an evolved gas analysis furnace. A ThermoNicolet Nexus FTIR bench, equipped with FTIR cell gas analysis, is coupled to TGA apparatus *via* a transfer line.

A sample is placed in an opened aluminum basket and analyzed according to conditions described in Table 6.

**Table 6: Operating conditions for TGA and TGA-FTIR analysis**

| | | **TGA** | **TGA-FTIR** |
|---|---|---|---|
| *TGA apparatus* | | | |
| Mass assay (mg) | | #5 | # 5 |
| Heating ramp (°C/min) | | +10 | +10 |
| Final temperature (°C) | | +500 | +200 |
| Carrier gas | - nitrogen | Messer, "Nitrogen quality 5.0" | |
| | - flow rate (mL/min) | 95-105 | |

| *FTIR bench* | | | |
|---|---|---|---|
| Transfer line temperature (°C) | | - | +250 |
| FTIR cell gas analysis temperature (°C) | | - | +250 |
| Beam splitter | | - | KBr |
| Detector | | - | DTGS |
| Spectral resolution (cm⁻¹) | | - | 4 |
| Mirror speed (cm.s⁻¹) | | - | 2.5317 |
| Bench purge gas | | - | Dry air |

### Results:

TGA and DSC profiles are respectively displayed in Figure 2 and Figure 3.

TGA and DSC detailed integration results are respectively presented in Table 7 and Table 8.

On the TGA trace, upon heating, no mass loss is detected before the main thermal decomposition detected with an onset temperature of +333°C.

On the DSC trace, the strong and sharp endotherm is detected with an onset temperature of +172°C which is attributed to the melting of the sample.

**Table 7 : Quantitative results for TGA experiment for crystalline form I**

| Step | **Mass loss upon heating** | | | | |
|---|---|---|---|---|---|
| | Δm (%) | Start (°C) | T_{Onset} (°C) | T_{Endset} (°C) | Stop (°C) |
| 1 | 99.5 | +183 | +333 | +377 | +486 |

**Table 8 : Quantitative DSC results for crystalline Form I**

| **Event** | **Transition** | **Temperature (°C)** | | **Enthalpy transition** |
|---|---|---|---|---|
| | | Onset | Peak | (J/g) |
| 1 | Strong endotherm (melting) | +172 | +174 | -101 |

### II.3. Dynamic water vapor sorption (DVS) analysis.

### Method:

The dynamic vapor sorption (DVS) analysis (gas water sorption) is performed on a DVS-Intrinsic incubator (SMS Ltd), equipped with DVS-Intrinsic Control Software 1.0.

The sample, placed in an aluminum basket holder, is submitted to a full-cycle analysis (sorption followed by desorption cycle) under the conditions described in Table 9.

The sample is first pre-dried under a stream of dry air until a stabilized sample mass is observed. Then, the relative humidity is increased by 5 % increments. At each step, the sample mass is allowed to increase until equilibrium is reached (according to a given dm/dt target), and then a new relative humidity rise occurs. Relative humidity is ramped up to 95 %. After equilibration at this point, desorption begins in a similar stepwise manner, with sample mass decrease again allowed to stabilize after each incremental humidity decrease.

With both segments of the above described method, the sample mass recording allows describing of the whole vapor water sorption/desorption behavior *versus* relative humidity.

**Table 9 : Operating conditions for water vapor sorption/desorption analysis**

| | |
|---|---|
| Temperature | +25°C |
| Carrier gas and rate | dried and filtered air at 200 mL.min⁻¹ |
| Mode and criterion | dm/dt ≤ 0.002%.min⁻¹ |
| Humidity range | 0 to 95% RH |
| RH step | 5% |
| Minimum step time | 10 minutes |
| Maximum step time | 360 minutes |

### Results:

*Preliminary drying:* The sample is first dried (maintained at 0% RH) before being submitted to the sorption-desorption cycle. This allows assessing the potential amount of water initially present in the bulk.

During the preliminary drying phase (sample maintained at +25°C/0% RH before starting the vapor water sorption process, see Figure 4), the sample loses only 0.12% of its mass.

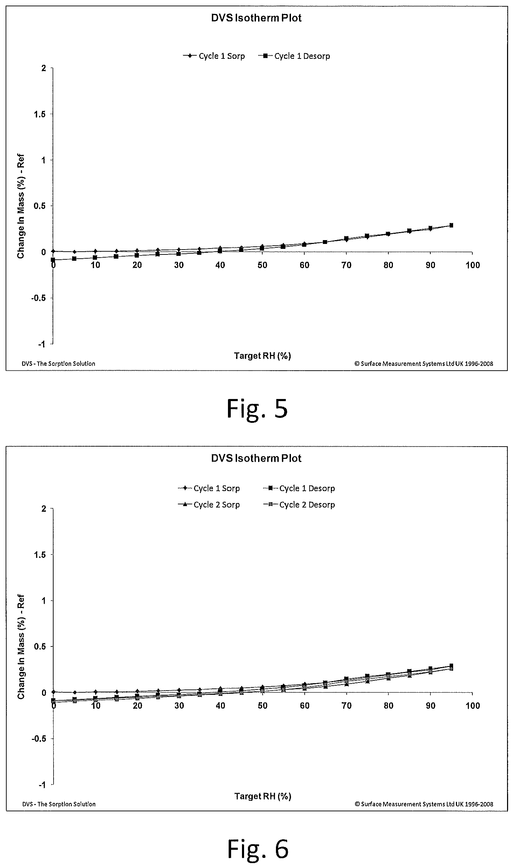

Figure 5 then displays the DVS isotherm plots (water vapor sorption/desorption traces at +25°C) for the tested sample of the crystalline form I.

*Upon sorption,* three relative humidity (RH) intervals corresponding to different water sorption rates can be observed for the sample mass variation:
- 0% to 10 % RH: no significant mass variation of the sample is observed.
- 10% to 60% RH: a very slow and regular increase of the sample mass is observed, corresponding to water sorption onto the bulk. Water uptake at +25°C/60% RH is 0.09%.
- 60% to 95% RH: a change in the water adsorption rate is observed with a very slighter increase of the sample mass. Total water uptake at +25°C/95% RH is only 0.29%.

When submitted to a decreasing relative humidity, the test sample loses its adsorbed water without hysteresis between 95% and 65% RH.

From 65% to 0% RH, the test sample loses even a little bit more, the final sample mass being 0.09% lower than the starting mass (after pre-drying before 1^{st} sorption).

With its 0.09% water uptake at 60% RH, the studied crystalline form I can be considered as being non-hygroscopic at all (compound bulks are usually said to start being hygroscopic above 2% mass gain at +25°C/60% RH) and does not convert into any hydrated form, even when submitted to high relative humidity up to 95%.

The sample resulting from this first DVS cycle (1^{st} sorption + 1^{st} desorption) is then submitted to a second sorption/desorption cycle.

A similar water sorption/desorption behavior than the one observed during the first sorption/desorption cycle is then observed but this time the sorption/desorption curves are perfectly overlaid (see Figure 6).

When coming back to 0% RH after the second sorption/desorption cycle, the final sample mass is the same as what it was at the end of the first sorption/desorption cycle.

### III. Crystalline forms obtained with the 6 other solvents.

The crystalline Form I i.e. the crystalline form of 2-(4-chlorobenzylamino)-4-(4-*tert*-butylaminopiperidin-1-yl)-quinoline obtained in ethanol is identified in the other 6 medias (Acetone, Methyl Ethyl Ketone, Ethyl Acetate, Propanol-1, isopropyl Acetate and Propanol-2). All these samples indeed present the same XRPD and DSC patterns as the crystalline Form !.

### IV. Powder stability under temperature and humidity stress of crystalline form I.

The chemical and physical accelerated stability of crystalline form I (see example II) is studied in order to anticipate potential stability issues upon storage or ageing.

Crystalline form I powder is stored for 1 month at +40°C/75%RH and is also stored for 1 month at +60°C (with %RH < 10 %).

The chemical stability is then assessed by chromatography by external standardization *versus* a freshly prepared (non-stressed crystalline form I) standard solution. For that purpose, three samples (exactly weighted) are stored for each condition.

The physical stability is also assessed by XRPD, DSC and TGA analyses by comparing the XRPD and thermal profiles of stressed samples to those of a non-stressed sample. For that purpose, one more sample is stored in each condition (respectively +40°C/75% RH and +60°C).

### IV.1. Chemical stability.

### Method: High Performance Liquid Chromatography (HPLC) method

**Table 10 : HPLC conditions**

| | | | |
|---|---|---|---|
| HPLC system | Injector / Pump: Alliance 2695 Waters | | |
| | Detector: Photo Diode Array 996 Waters | | |
| | Software: Empower Waters | | |
| Column | Symmetry Shield RP18 | | |
| | 150 mm x 4.6 mm - dp = 5 µm | | |
| Mobile phase | A: H₂O/TFA 0.05% | | |
| | B: CH₃CN/TFA 0.05% | | |
| | Time (min) | A % | B % |
| | 0 | 95 | 5 |
| | 1 | 95 | 5 |
| | 9 | 10 | 90 |
| | 12 | 10 | 90 |
| | 12.1 | 95 | 5 |
| | 15 | 95 | 5 |
| Flow rate | 1 mL/min | | |
| Column Temperature | Room temperature | | |
| Detection | UV: λ = 323 nm | | |
| Standard solution | T100%: ≈ 2.5 mg of crystalline form I of compound I-b, qs 25 mL with H₂O/CH₃CN 20/80 (v/v) | | |
| | 3 independent standard solutions are performed | | |
| Test solution | Suitable dilution in H₂O/CH₃CN 20/80 (v/v) | | |
| | 3 independent assays are performed | | |
| Injection volume | 10 µL | | |
| Injector temperature | + 20°C | | |
| Retention time | ≈ 6.4 min for compound I-b | | |

*Result:* The results of the HPLC analyses are displayed in Table 11.

**Table 11 : Crystalline Form I chemical degradation assessment by HPLC after temperature and humidity stress**

| **Sample** | **% of unchanged compound left** *(HPLC-UV at 323 nm)* | **Impurity profile compared to the standard profile** *(HPLC-UV at 323 nm, 252 nm and 203 nm)* |
|---|---|---|
| 1 month +40°C/75%RH | 99.7 (%CV=0.2 % on 3 independent assays) | Similar to standard profile (% *purity* = *98.7* % *at 323 nm)* |
| | | No additional impurity |
| 1 month +60°C | 100.5 (%CV=0.1 % on 3 independent assays) | Similar to standard profile (% *purity* = *98.8* % *at 323 nm)* |
| | | No additional impurity |
| % of unchanged compound left is determined *versus* standard solutions | | |
| *Container: open (only for 40°C*/*75%RH storage) glass vials protected from light* | | |

Given the above results, crystalline form I can be considered to be chemically stable as a bulk powder, at least 1-month after storage at +40°C/75%RH and at +60°C.

### IV.2. Physical stability results.

Table 12, Table 13 and Table 14 display the physical characterization results of the bulk sample stored for 1 month at +40°C/75%RH and at +60°C compared to the initial characterization.

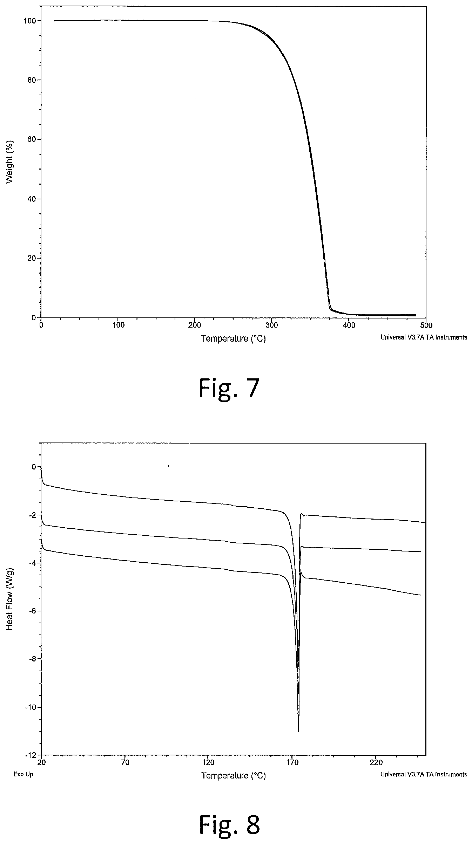

Figure 7, Figure 8 and Figure 9 respectively display overlaid TGA profiles, overlaid DSC profiles and overlaid XRPD patterns of a non-stressed sample and of samples stored for 1 month at +40°C/75%RH and at +60°C.

### TGA profiles:

**Table 12 : Crystalline Form I physical stability assessment by TGA after temperature and humidity stress**

| **Sample** | **Step** | **Mass losses upon heating** | | | | |
|---|---|---|---|---|---|---|
| | | Δm (%) | Start (°C) | T_{Onset} (°C) | T_{Endset} (°C) | Stop (°C) |
| Initial analysis | 1 | 99.5 | +183 | +333 | +377 | +486 |
| 1 month +40°C/75% RH | 1 | 99.2 | +179 | +333 | +375 | +486 |
| 1 month +60°C | 1 | 99.4 | +180 | +334 | +377 | +486 |

No difference is evidenced by TGA analysis between the stressed samples and the initial physical form of the bulk (Figure 7). For all samples, no mass loss is detected before the main thermal decomposition detected with an onset temperature of +333/+334°C.

### DSC profiles:

**Table 13 : Crystalline Form I physical stability assessment by DSC after temperature and humidity stress**

| **Sample** | **Event** | **Transition** | **Temperature (°C)** | | **Enthalpy transition** |
|---|---|---|---|---|---|
| | | | Onset | Peak | (J/g) |
| Initial analysis | 1 | Strong endotherm (melting) | +172 | +174 | -101 |
| 2 weeks 40°C/75%RH | 1 | Strong endotherm (melting) | +172 | +174 | -102 |
| 2 weeks 60°C | 1 | Strong endotherm (melting) | +172 | +174 | -104 |

No DSC profile difference is evidenced between stressed samples and initial physical form (Figure 8).

### XRPD patterns:

**Table 14 : Crystalline Form I physical stability assessment by XRPD after temperature and humidity stress**

| **Sample** | **XRPD results** |
|---|---|
| Initial analysis | Crystalline material (several sharp and intense diffraction peaks present on the diffractogram) |
| 1 month +40°C/75%HR | Diffractogram comparable to the initial diffractogram |
| 1 month +60°C | Diffractogram comparable to the initial diffractogram |

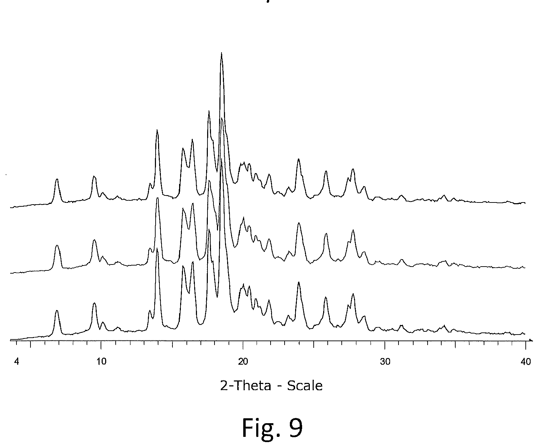

No XRPD pattern difference is evidenced between stressed samples and initial physical form (Figure 9).

### Conclusion:

Crystalline Form I can be considered to be physically stable as a bulk powder, at least 1 month after storage at +40°C/75% HR and at +60°C.

## Claims

1. A crystalline freebase form of 2-(4-chlorobenzylamino)-4-(4-tert-butylaminopiperidin-1-yl)-quinoline **characterized by** a X-ray powder diffraction pattern comprising the diffraction peaks at 2θ values of 13.9±0.1, 16.4±0.1, 17.5±0.1, 17.9±0.1, 18.5±0.1, and 18.8±0.1.

2. The crystalline freebase form according to claim 1, wherein said X-ray powder diffraction pattern further comprises at least one diffraction peak at 2θ values selected in the group consisting of 9.5±0.1, 15.7±0.1, 19.8±0.1, 20.0±0.1, 20.4±0.1, 20.9±0.1, 21.9±0.1, 24.0±0.1, 24.2±0.1, 25.8±0.1, and 27.8±0.1.

3. The crystalline freebase form according to claim 2, wherein said X-ray powder diffraction pattern further comprises at least one diffraction peak at 2θ values selected in 6.8±0.1, 13.4±0.1, 21.2±0.1, 23.3±0.1, 27.4±0.1, and 28.6±0.1.

4. The crystalline freebase form according to any one of claims 1 to 3, wherein said X-ray powder diffraction pattern (XRPD) is as shown in Figure 1.

5. The crystalline freebase form according to any one of claims 1 to 4, wherein said crystalline freebase form is **characterized by** the thermogravimetric analysis (TGA) trace of Figure 2.

6. The crystalline freebase form according to any one of claims 1 to 5, wherein said crystalline freebase form is **characterized by** the Differential Scanning Calorimetry (DSC) thermograph of Figure 3 which shows a melting point of about +172°C.

7. Process for preparing the crystalline freebase form as defined in any one of claims 1 to 6, the process comprising the steps of:
a) mixing 2-(4-chlorobenzylamino)-4-(4-tert-butylaminopiperidin-1-yl)-quinoline with a solvent selected from the group consisting of ethanol, acetone, methyl ethyl ketone, ethyl acetate, propanol-1, isopropyl acetate and propanol-2;
b) heating the mixture to refluxing conditions or until complete dissolution of 2-(4-chlorobenzylamino)-4-(4-*tert*-butylaminopiperidin-1-yl)-quinoline;
c) cooling the mixture to a temperature below or equal to room temperature (22°C ± 5°C) and
d) isolating the crystalline freebase Form I from the mixture.

8. Preparation process according to claim 7, wherein, during said step c), crystalline freebase form as defined in any one of claims 1 to 6 can be added to the mixture.

9. A pharmaceutical composition comprising, as active ingredient, the crystalline freebase form according to any one of claims 1 to 6 and at least one pharmaceutically acceptable vehicle.

10. The pharmaceutical composition according to claim 9, further comprising at least one additional therapeutic agent.

11. The pharmaceutical composition according to claim 10, wherein said at least one additional therapeutic agent is selected in the group consisting of anti-neoplastic agents, agents used to palliate, ameliorate, stabilize, reverse, slow or delay the progression of the cancer state and/or cancer related diseases; anti-fibrotic agents, agents used to palliate, ameliorate, stabilize, reverse, slow or delay the progression of the fibrotic state; anti-autophagic agents or agents used to inhibit the autophagy flux, agents used to palliate, ameliorate, stabilize, reverse, slow or delay the progression of the autophagic disease state and/or related diseases ; inhibitors of cathepsins B (CTSB), L (CTSL) and/or D (CTSD), and agents used to palliate, ameliorate, stabilize, reverse, slow or delay the progression of cathepsins B (CTSB), L (CTSL) and/or D (CTSD) related diseases.

12. A kit of parts comprising or consisting of:
a) the crystalline freebase form as defined in any one of claims 1 to 6 or a pharmaceutical composition as defined in any one of claims 9 to 11, and
b) at least one additional therapeutic agent or a pharmaceutical composition comprising at least one additional therapeutic agent.

13. The crystalline freebase form according to any one of claims 1 to 6, the pharmaceutical composition according to any one of claims 9 to 11 and/or the kit of parts according to claim 12 for use in preventing and/or treating a proliferative and/or neoplastic disease.

14. The crystalline freebase form, the pharmaceutical composition and/or the kit of parts for use according to claim 13, wherein the proliferative and/or neoplastic disease is a cancer related disease selected in the group consisting of a lung cancer, an ovary cancer, a pancreas cancer, a hepatocarcinoma, a hepatoblastoma and a cholangiocarcinoma such as an intrahepatic cholangiocarcinoma or an extrahepatic cholangiocarcinoma.

15. The crystalline freebase form, the pharmaceutical composition and/or the kit of parts for use according to claim 13, wherein the proliferative and/or neoplastic disease is a liver cancer related disease.

16. The crystalline freebase form, the pharmaceutical composition and/or the kit of parts for use according to claim 15, wherein the liver cancer related disease is a hepatocarcinoma.

17. The crystalline freebase form, the pharmaceutical composition and/or the kit of parts for use according to claim 15, wherein the liver cancer related disease is a cholangiocarcinoma such as an intrahepatic cholangiocarcinoma or an extrahepatic cholangiocarcinoma.

18. The crystalline freebase form according to any one of claims 1 to 6, the pharmaceutical composition according to any one of claims 9 to 11 and/or the kit of parts according to claim 12 for use in inhibiting the growth or differentiation of a Cancer Stem Cell (CSC), a tumor initiating cell, a mesenchymal-like cell associated with cancer, a mesenchymal cancerous cell, or a mesenchymal cell.

19. The crystalline freebase form according to any one of claims 1 to 6, the pharmaceutical composition according to any one of claims 9 to 11 and/or the kit of parts according to claim 12 for use in treating and/or decreasing and/or preventing fibrosis and/or fibrosis related diseases.

20. The crystalline freebase form according to any one of claims 1 to 6, the pharmaceutical composition according to any one of claims 9 to 11 and/or the kit of parts according to claim 12 for use in decreasing and/or preventing the autophagy and/or autophagy related diseases and inhibiting autophagy flux related diseases.

21. The crystalline freebase form according to any one of claims 1 to 6, the pharmaceutical composition according to any one of claims 9 to 11 and/or the kit of parts according to claim 12 for use in inhibiting cathepsins B (CTSB), L (CTSL) and/or D (CTSD) and/or treating and/or preventing cathepsins B (CTSB), L (CTSL) and/or D (CTSD) related diseases.
